# EUROPEAN PATENT APPLICATION

(11) **EP 3 909 542 A2**
(43) Date of publication of application: **17.11.2021**
(21) Application number: 21173704.4
(22) Date of filing: 12.05.2021
(51) Int. Cl.: A61B 90/98, A61B 17/00

(54) **SURGICAL SYSTEMS AND METHODS FOR PROTECTING AGAINST UNAUTHORIZED USE**

(30) Priority: 12.05.2020 US 202063023383 P; 05.05.2021 US 202117308924
(71) Applicant: Covidien LP, Mansfield, MA 02048 (US)
(72) Inventor: BEGG, Nikolai, D, Woburn, MA 01801 (US); MARINKOVIC, Aleksandar, Woburn, MA 01801 (US); GEARHEART, John, R, Woburn, MA 01801 (US)
(74) Representative: Maschio, Antonio

(57) **Abstract**

A surgical device includes a limited-use component configured to perform a surgical operation, where the limited-use component can be used for a pre-determined number of uses, and a reusable component operationally coupled to the limited-use component. The limited-use component includes a body forming a pocket and a radio frequency identification (RFID) tag disposed within the pocket. The reusable component includes an RFID tag transceiver configured to read operating parameters from a memory of the RFID tag of the limited-use component. The operating parameters are erased from the memory when the limited-use component has been used for the pre-determined number of uses.

## Description

### CROSS-REFERENCE TO RELATED APPLICATIONS

This application claims the benefit of, and priority to, U.S. Provisional Patent Application No. 63/023,383, filed on May 12, 2020, the entire contents of which are hereby incorporated herein by reference.

### FIELD

The present disclosure is generally related to surgical systems and methods for protecting against unauthorized use and, more particularly, to surgical systems for using radio frequency identification (RFID) to prevent reuse of a limited use device.

### BACKGROUND

Surgical devices intended for single- or limited-use may be inappropriately reprocessed and reused in a manner contrary to the manufacturer's specifications. Unauthorized use of such inappropriately reprocessed devices raises a variety of safety issues not present when a device is used the authorized number of times as permitted by the manufacturer.

### SUMMARY

This disclosure generally relates to protecting against unauthorized use of a surgical device that includes a limited-use component. Based on information stored on an RFID tag in the limited-use component, the surgical device becomes unavailable for continued use after the authorized number of uses of the limited-use component is reached.

Provided in accordance with aspects of the present disclosure is a surgical device to perform a surgical operation. The surgical device includes a limited-use component configured to perform a surgical operation, where the limited-use component can be used for a pre-determined number of uses, and a reusable component operationally coupled to the limited-use component. The limited-use component includes a body forming a pocket and a radio frequency identification (RFID) tag disposed within the pocket. The reusable component includes an RFID tag transceiver configured to read operating parameters from a memory of the RFID tag of the limited-use component. The operating parameters are erased from the memory when the limited-use component has been used for the pre-determined number of uses.

In an aspect of the present disclosure, the RFID tag transceiver may be disposed in vertical registration with the RFID tag when the reusable component is operationally coupled to the limited-use component.

In another aspect of the present disclosure, the operating parameters may be encrypted to limit reproduction and reprograming of the operating parameters.

In still another aspect of the present disclosure, the operating parameters may include at least one of a home/initial position of the limited-use component, a rotation type, revolution per minute (RPM) settings, a maximum RPM, pressure setting information, vacuum setting information, outflow setting information, or calibration information.

Provided in accordance with aspects of the present disclosure is a surgical device to perform a surgical operation. The surgical device includes a limited-use component configured to perform a surgical operation, where the limited-use component can be used for a pre-determined number of uses. The limited-use component includes a body forming a pocket, a radio frequency identification (RFID) tag disposed within the pocket, and an irreversible electrical component electrically coupled to the RFID tag. The surgical device further includes a reusable component operationally coupled to the limited-use component. The reusable component includes an RFID tag transceiver configured to read operating parameters from a memory of the RFID tag of the limited-use component.

In an aspect of the present disclosure, activation of the memory may include the RFID tag transceiver writing new information in the memory and immediately reading the new information from the memory.

In another aspect of the present disclosure, the RFID tag transceiver may be disposed in vertical registration with the RFID tag when the reusable component is operationally coupled to the limited-use component.

In yet another aspect of the present disclosure, the operating parameters may be encrypted to limit reproduction and reprograming of the operating parameters.

In still yet another aspect of the present disclosure, the operating parameters may include at least one of a home/initial position of the limited-use component, a rotation type, revolution per minute (RPM) settings, a maximum RPM, pressure setting information, vacuum setting information, outflow setting information, or calibration information.

Provided in accordance with aspects of the present disclosure is a surgical device to perform a surgical operation. The surgical device includes a limited-use component configured to perform a surgical operation, where the limited-use component can be used for a pre-determined number of uses. The limited-use component includes a body forming a pocket, a radio frequency identification (RFID) tag disposed within the pocket, and an irreversible electrical component electrically coupled to the RFID tag. The surgical device further includes a reusable component operationally coupled to the limited-use component. The reusable component includes an RFID tag transceiver configured to read operating parameters from a memory of the RFID tag of the limited-use component.

In an aspect of the present disclosure, the irreversible electrical component may be activated when the limited-use component has been used for the predetermined number of uses.

In another aspect of the present disclosure, the irreversible electrical component may be a fuse.

In still another aspect of the present disclosure, the RFID tag transceiver may be disposed in vertical registration with the RFID tag when the reusable component is operationally coupled to the limited-use component.

In still another aspect of the present disclosure, the operating parameters may be encrypted to limit reproduction and reprograming of the operating parameters.

In still yet another aspect of the present disclosure, the operating parameters may include at least one of a home/initial position of the limited-use component, a rotation type, revolution per minute (RPM) settings, a maximum RPM, pressure setting information, vacuum setting information, outflow setting information, or calibration information.

### BRIEF DESCRIPTION OF THE DRAWINGS

Various aspects and features of the present disclosure are described hereinbelow with reference to the drawings wherein like numerals designate identical or corresponding elements in each of the several views.
FIG. 1 is a perspective view of a surgical system including different types of surgical devices in accordance with aspects of the present disclosure;
FIG. 2 is a side, perspective view of a tissue resecting instrument including an elongated probe engaged with a handpiece assembly in accordance with aspects of the present disclosure;
FIG. 3 is a side, perspective, exploded view of the end effector assembly of the tissue resecting instrument of FIG. 2 in accordance with aspects of the present disclosure;
FIG. 4 is a side, perspective view of the proximal end portion of the end effector assembly of the tissue resecting instrument of FIG. 2 in accordance with aspects of the present disclosure;
FIG. 5 is a longitudinal, cross-sectional view taken across section line "5-5" of FIG. 4 in accordance with aspects of the present disclosure; and
FIG. 6 is a longitudinal, cross-sectional view taken across section line "6-6" of FIG. 2 in accordance with aspects of the present disclosure.

### DETAILED DESCRIPTION

Surgical devices may include one or more reusable, single-use, and/or limited-use components. By limiting use of certain components to a one-time (single) use or to a predetermined number of times (limited) use, a manufacturer can ensure a certain level of performance thereof. Radio frequency identification (RFID) may be used in accordance with the present disclosure to limit the number of uses of such components. Provided herein are surgical devices, which include one or more single-use or limited-use components, and which incorporate RFID technology to prevent unauthorized uses more than an allowed number of uses.

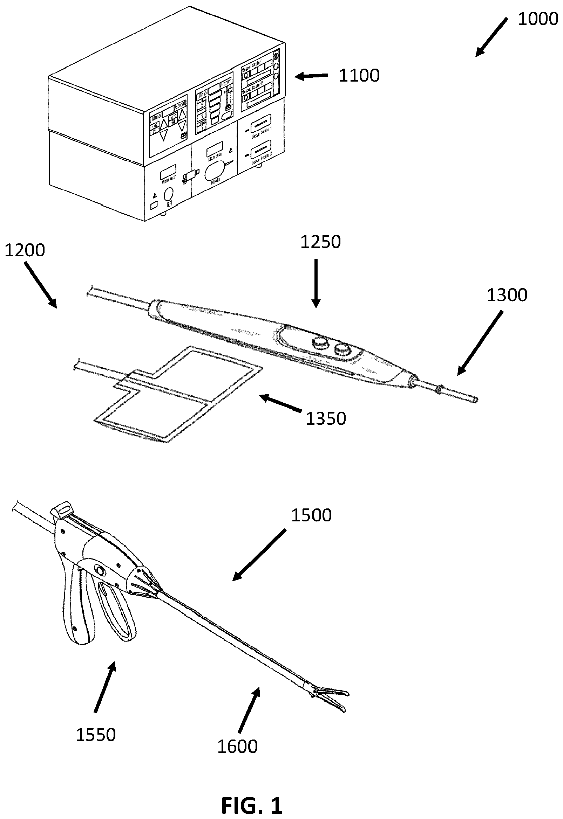

Referring to FIG. 1, a surgical system 1000 may include a surgical generator 1100, a monopolar surgical device 1200, and/or a bipolar surgical device 1500. The monopolar surgical device 1200 may have a handpiece assembly 1250, an elongated probe or end effector assembly 1300 for treating tissue of the patient (e.g., cutting, ablating, sealing, etc.), and a return pad 1350. The elongated probe 1300 may be for a one-time use and the handpiece assembly 1250 may be reusable. By replacing the elongated probe 1300, which contacts the tissue of interest, potential contamination caused by reuse of the elongated probe 1300 may be avoided, and one-time use of the elongated probe 1300 may avoid deterioration of functionality that may occur due to over-usage.

The monopolar surgical device 1200 may be connected to the surgical generator 1100 via one of several output connectors thereof. The surgical generator 1100 generates surgical energy in the form of radio frequency (RF) energy, although other suitable energies, e.g., thermal, microwave, ultrasonic, light, etc., are also contemplated. The surgical energy is supplied to the monopolar surgical device 1200, which applies the surgical energy to treat the tissue via the elongated probe 1300. The surgical energy is then returned to the surgical generator 1100 through the return pad 1350. The return pad 1350 provides a sufficient contact area with the patient's tissue so as to minimize the risk of tissue damages due to the surgical energy applied to the tissue.

The bipolar surgical device 1500 may include a handpiece assembly 1550 and an elongated probe or end effector assembly 1600. The handpiece assembly 1550 may be reusable and the elongated probe 1600 may be configured for one-time use. The bipolar surgical device 1500 can be also connected to the surgical generator 1100 via one of several output connectors. The surgical energy is supplied to one of the two jaw members of the elongated probe 1600 to treat the tissue and is returned to the surgical generator 1100 through the other one of the two jaw members, although other energy modalities are also contemplated such as those noted above.

The surgical generator 1100 may be any suitable type of generator and may include a plurality of connectors to accommodate various types of surgical devices (e.g., monopolar surgical device 1200 and bipolar surgical device 1500). The surgical generator 1100 may also be configured to operate in a variety of modes, such as ablation, cutting, coagulation, and sealing. The surgical generator 1100 may include a switching mechanism (e.g., relays) to switch the supply of RF energy among the connectors to which various surgical devices may be connected. For example, when the monopolar surgical device 1200 is connected to the surgical generator 1100, the switching mechanism switches the supply of RF energy to the monopolar plug. In aspects, the surgical generator 1100 may be configured to provide RF energy to a plurality of surgical devices simultaneously.

The surgical generator 1100 includes a user interface having suitable user controls (e.g., buttons, activators, switches, or touch screens) for providing control parameters to the surgical generator 1100. These controls allow the user to adjust parameters of the surgical energy (e.g., the power level or the shape of the output waveform) so that the surgical energy is suitable for a particular surgical procedure (e.g., coagulating, ablating, sealing, or cutting). The energy delivery devices 1200 and 1500 may also include a plurality of user controls. In addition, the surgical generator 1100 may include one or more display screens for displaying a variety of information related to operation of the surgical generator 1100 (e.g., intensity settings and treatment complete indicators).

In aspects, the surgical system 1000 may be a robotic system and the monopolar and bipolar surgical devices 1200, 1500 may be connected to the surgical generator 1100 via a robotic device, e.g., a robotic arm.

The reusable component 1250 or 1550 of the monopolar or bipolar surgical device 1200 or 1500 may be capable of communicating with the limited-use component 1300 or 1600 to make sure that the limited-use component 1300 or 1600 can be used once or up to a predetermined or authorized number of times.

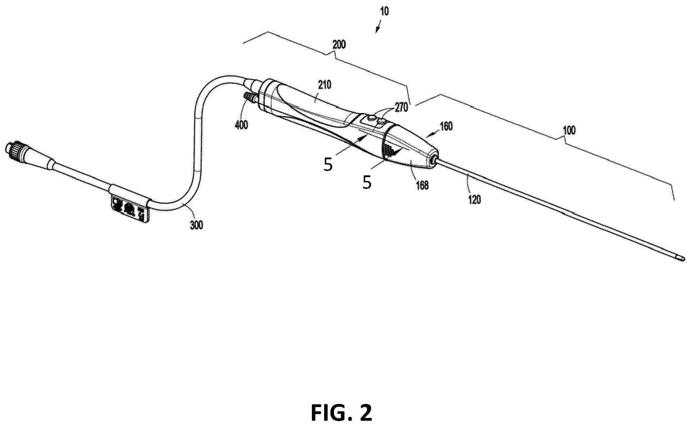

When the limited-use component 1300 or 1600 is used, the reusable component 1250 or 1550 counts the number of uses of the limited-use component 1250 or 1550 and save the usage number in a memory of the limited-use component 1250 or 1550. When the number of uses reaches the authorized/predetermined/approved number, the limited-use component 1250 or 1550 cannot be used further. In this way, the limited-use component 1250 or 1550 may be used up to the approved/authorized number of times. Further, the limited-use component 1250 or 1550 may store the authorized or approved number of usages in the memory. Details regarding mechanisms to deactivate the limited-use component 1250 or 1550 will be described below with reference to FIGS. 2-4.

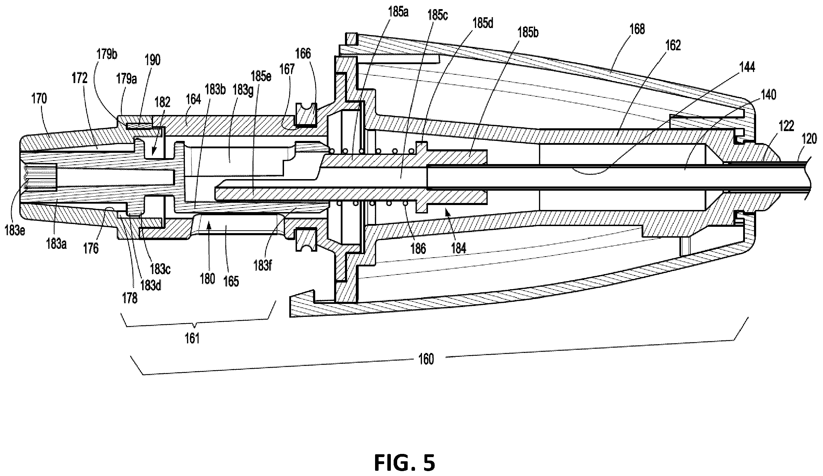

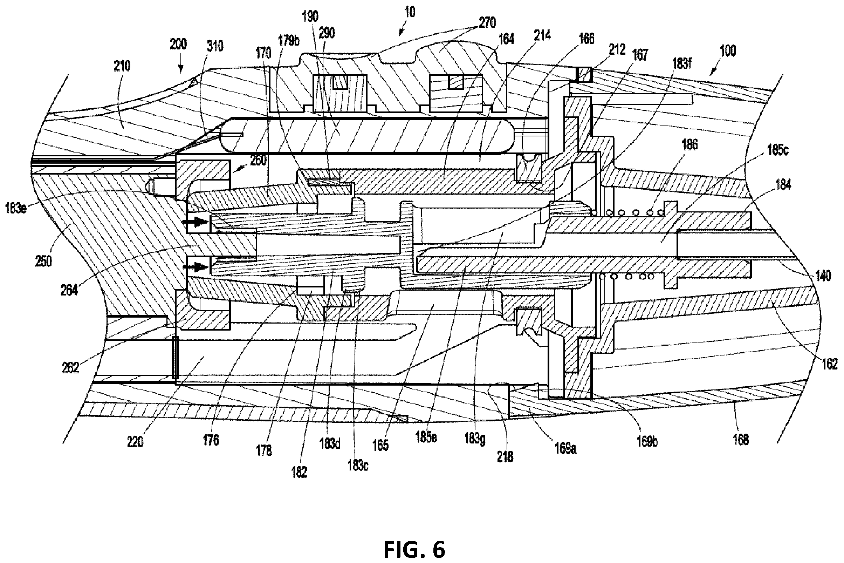

Referring generally to FIGS. 2-6, another surgical instrument 10 is provided in accordance with the present disclosure and configured to resect tissue. Surgical instrument 10 includes an end effector assembly 100 and a handpiece assembly 200. End effector assembly 100 is detailed below as incorporating an RFID chip 190. It is understood that RFID chip 190 may similarly be incorporated into the limited-use component 1250 or 1550 of device 1200 or 1500, or other suitable component of a surgical device for similar purposes.

The surgical instrument 10 is adapted to connect to a control unit (not shown) via a cable 300 to provide power and control functionality to the surgical instrument 10, although the surgical instrument 10 may alternatively or additionally include a power source, e.g., battery, and/or a control unit disposed within the handpiece assembly 200. The surgical instrument 10 is further adapted to connect to a fluid management system (not shown) via outflow tubing (not shown) connected to outflow port 400 for applying suction to remove fluid, tissue, and debris from a surgical site via the surgical instrument 10. The control unit and fluid management system may be integral with one another, coupled to one another, or separate from one another.

The end effector assembly 100 of the surgical instrument 10 may be configured as a single-use (or limited-use) device that is discarded after use (or uses) for repeated use by the end-user, or a partially-single-use, partially-reusable device. With respect to partially-single-use, partially reusable configurations, the handpiece assembly 200 may be configured as a cleanable/sterilizable, reusable component, while the end effector assembly 100 is configured as a single-use, disposable/reprocessable component. In any of the above configurations, the end effector assembly 100 is configured to releasably engage the handpiece assembly 200 to facilitate disposal/reprocessing of any single-use components and cleaning and/or sterilization of any reusable components. Further, enabling releasable engagement of the end effector assembly 100 with the handpiece assembly 200 allows for interchangeable use of different end effector assemblies, e.g., different length, configuration, etc., end effector assemblies, with the handpiece assembly 200.

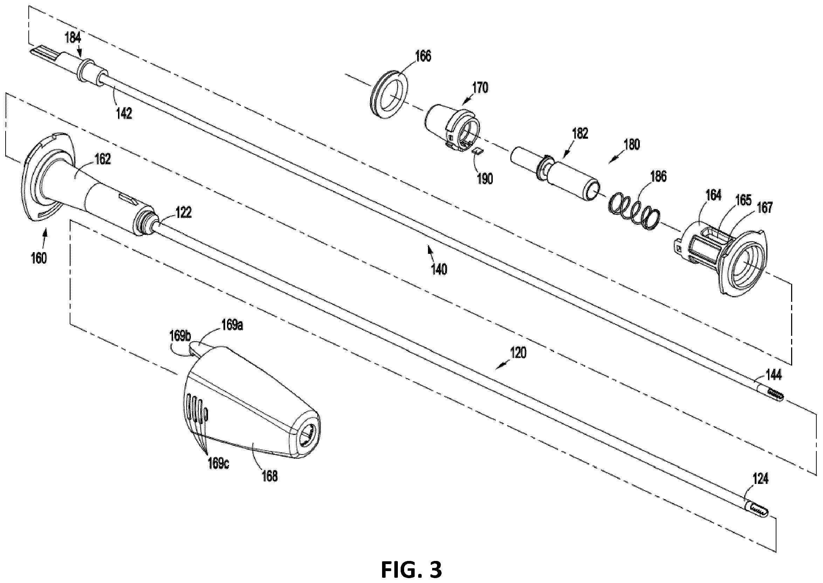

The end effector assembly 100 may include an outer shaft 120, an inner shaft 140, a hub assembly 160, a drive assembly 180, and an RFID chip 190, as shown in FIG. 3. The outer shaft 120 includes a proximal end portion 122 and a distal end portion 124 defining at least a partially closed distal end.

The inner shaft 140 is rotatably disposed within the outer shaft 120 and includes a proximal end portion 142 and a distal end portion 144 defining at least a partially closed distal end 146. The inner shaft 140 is configured for rotation and/or oscillation within and relative to the outer shaft 120 to facilitate intended surgical operations of the end effector assembly 100.

The inner shaft 140 may be driven to rotate continuously in a single direction. Alternatively, inner shaft 140 may be configured to repeatedly oscillate, rotating in one direction and then rotating in the other direction. The end effector assembly 100 may be driven in either the rotational or oscillatory fashion, depending upon the input received from the handpiece assembly 200. Translational motion, in addition or as an alternative to rotational motion, is also contemplated.

As noted above, the end effector assembly 100 includes the outer shaft 120, the inner shaft 140, the hub assembly 160, and the drive assembly 180. The end effector assembly 100 may further include an RFID chip 190 captured between a retainer cap 170 of the hub assembly 160 and a proximal extension portion 164 of a hub housing 161 of the hub assembly 160.

The RFID chip 190 may be used to limit the number of uses of the end effector assembly 100. In particular, the RFID chip 190 may include a memory, which stores information of the end effector assembly 100 and operating parameters of the end effector assembly 100.

In an aspect, the operating parameters may be encrypted to limit reproduction and reprogramming of the end effector assembly 100 to protect against tampering with the memory of the RFID chip 190. Further, encryption may protect the limitation on the number of uses of the end effector assembly 100.

The hub assembly 160 includes a hub housing 161 having a distal body portion 162 and a proximal extension portion 164 that are configured for engagement with one another, e.g., via snap-fitting or other suitable engagement. With the end effector assembly 100 engaged with the handpiece assembly 200, the proximal extension portion 164 of the hub housing 161 extends into the handpiece assembly 200 while the distal body portion 162 substantially abuts and extends distally from the handpiece assembly 200. The proximal extension portion 164 of the hub housing 161 further defines an outflow opening 165 through a sidewall thereof that is configured to fluidly communicate with an internal bore 214 of the handle housing 210 of the handpiece assembly 200 when the end effector assembly 100 is engaged therewith.

The distal body portion 162 of the hub housing 161 is fixedly disposed about the proximal end portion 122 of the outer shaft 120 with the outer shaft 120 extending distally therefrom. The inner shaft 140 extends through the outer shaft 120, as noted above, and extends proximally through the distal body portion 162 of the hub housing 161 into the proximal extension portion 164 of the hub housing 161, thereby the drive assembly 180 is operably coupled to the proximal end portion 142 of the inner shaft 140.

The hub assembly 160 additionally includes an outer shell 168 configured for positioning about the distal body portion 162 of the hub housing 161 and for engagement therewith, e.g., via snap-fit engagement or in any other suitable manner. A cantilever engagement finger 169a extends proximally from a lower surface of the outer shell 168 of the hub housing 161 and proximally from the distal body portion 162 of the hub housing 161 when the outer shell 168 is engaged thereabout. The engagement finger 169a includes an engagement tooth 169b extending therefrom that is configured for engagement within an engagement aperture 218 defined within the handle housing 210 of the handpiece assembly 200 to enable releasable engagement of the end effector assembly 100 with the handpiece assembly 200. Grasping ribs 169c are defined on side surfaces of the outer shell 168 to facilitate engagement and disengagement of the end effector assembly 100 to and from the handpiece assembly 200.

With reference to FIG. 3, the retainer cap 170 of the hub assembly 160 is configured for snap-fit or other suitable engagement with a proximal end portion of the proximal extension portion 164. The retainer cap 170 defines a longitudinal lumen 174 extending through the retainer cap 170. An internal collar 176 protrudes radially inwardly into the longitudinal lumen 174. The internal collar 176 includes a distally-oriented notch 178 defined therein. The retainer cap 170 further includes an external collar defining a pocket 179b. The pocket 179b is configured to receive the RFID chip 190 therein. When the retainer cap 170 is engaged with the proximal extension portion 164, e.g., via snap-fitting, the open end of pocket 179b is blocked by a proximal face of the proximal extension portion 164, thereby securing the RFID chip 190 therein.

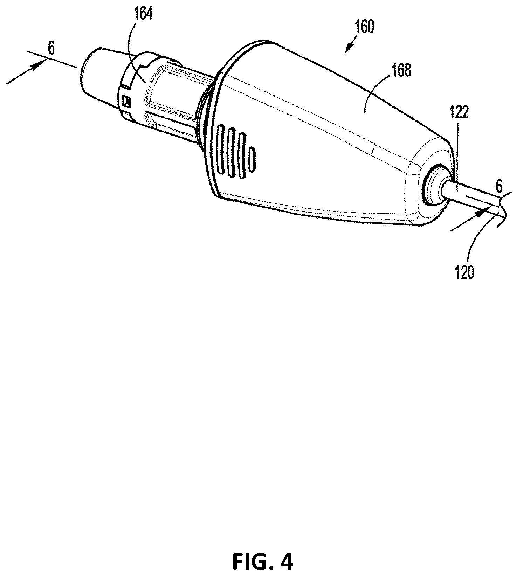

Referring generally to FIGS. 3 and 4, the drive assembly 180 is configured to operably couple a drive rotor 260 of the handpiece assembly 200 with the inner shaft 140 such that rotation of the drive rotor 260 drives rotation and/or oscillation of the inner shaft 140 within and relative to the outer shaft 120. The drive assembly 180, more specifically, includes a proximal driver 182, a distal driver 184, and a biasing spring 186, e.g., a coil compression spring. In some devices, the drive assembly 180 may further include a threaded coupler and cam follower (not shown) operable to convert rotation of the drive rotor 260 into reciprocation of the inner shaft 140 such that the inner shaft 140 is both rotated and reciprocated in response to rotation of the drive rotor 260. Additionally or alternatively, the drive assembly 180 may include gearing (not shown) configured to amplify or attenuate the output rotation of the inner shaft 140 relative to the input rotation from the drive rotor 260. Setting values or operating parameters of the inner shaft 140 of the end effector assembly 100 may be saved in the memory of the RFID chip 190.

The distal driver 184 of the drive assembly 180 is fixed about the proximal end portion 142 of the inner shaft 140 and includes a proximal body portion 185a, a distal body portion 185b, and a lumen 185c extending longitudinally therethrough. The distal driver 184 further includes a collar 186d disposed thereabout between the proximal and distal body portions 185a, 185b, respectively. The proximal body portion 185a of the distal driver 184 of the inner core drive assembly 150 includes a proximal foot 185e extending proximally therefrom. At least a portion of the proximal foot 184e defines a non-circular cross-sectional configuration, e.g., a semi-circular, rectangular or other polygonal configuration.

The RFID chip 190 is loaded into the pocket 179b of the retainer cap 170 and, thereafter, the retainer cap 170 is slid in a proximal-to-distal direction about the proximal driver 182 into engagement, e.g., via snap-fitting, with the proximal extension portion 164 of the hub housing 161. The internal collar 176 of the retainer cap 170 defines a diameter less than an outer diameter of the external collar 183c of the proximal body portion 183a of the proximal driver 182 such that the proximal driver 182 is inhibited from passing proximally therethrough. As a result, the engagement of the retainer cap 170 with the proximal extension portion 164 of the hub housing 161 retains the proximal driver 182 in engagement with the distal driver 184 against the bias of the biasing spring 186. Accordingly, once the retainer cap 170 is engaged with the proximal extension portion 164 of the hub housing 161, it is no longer required to hold the proximal driver 182.

In the fully assembled condition of the end effector assembly 100, as noted above, the biasing spring 186 biases the proximal driver 182 proximally such that the proximally-oriented tab 183d of the external collar 183c of the proximal body portion 183a of the proximal driver 182 is engaged within the distally-oriented notch 178 of the internal collar 176 of the retainer cap 170 to thereby rotationally fix the inner shaft 140 relative to the outer shaft 120. The end effector assembly 100, e.g., the proximal driver 182, the distal driver 184, and the retainer cap 170 thereof, may be configured such that, in the biased, rotationally locked position, corresponding to a closed position of the inner shaft 140 relative to the outer shaft 120.

The handpiece assembly 200 generally includes the handle housing 210, an outflow path 220 defined through the handle housing 210 and communicating with an outflow port 400, a motor 250 disposed within the handle housing 210, and a drive rotor 260 disposed within the handle housing 210 and operably coupled to the motor 250. The handpiece assembly 200 may further include one or more controls 270, e.g., buttons, disposed on the handle housing 210 to facilitate activation of the surgical instrument 10, toggle between various modes, and/or to vary the speed of the motor 250. Further, outflow tubing (not shown) is configured to connect to the outflow port 400 to thereby connect the outflow port 400 to a fluid management system (not shown). The fluid management system includes a vacuum source to establish suction through the surgical instrument 10 and the outflow tubing to facilitate removal of fluid, tissue, and debris from the surgical site and may also include a collection reservoir, e.g., a collection canister, for collecting the removed fluid, tissue, and debris. As an alternative or in addition to a vacuum source establishing suction through the surgical instrument 10 and the outflow tubing, vacuum may be created therethrough via a pressure differential between the surgical site and the outflow path.

The handle housing 210 defines a pencil-grip configuration, although other configurations are also contemplated, e.g., pistol-grip configurations, and includes an open distal end portion 212 communicating with an internal bore 214. The open distal end portion 212 of the handle housing 210 provides access to the drive rotor 260 and the internal bore 214 within the handle housing 210 such that, upon engagement of the end effector assembly 100 with the handpiece assembly 200, as detailed below, a portion of the end effector assembly 100 extends through the open distal end portion 212 and into the internal bore 214 to operably couple with the drive rotor 260 and fluidly couple the end effector assembly 100 with the internal bore 214 and, thus, the outflow path 220.

The cable 300 extends proximally from the handle housing 210 and is configured to connect to the control unit (not shown) to provide power and control functionality to the surgical instrument 10. The cable 300, more specifically, houses one or more wires (not shown) that extend into the handle housing 210 and electrically couple the controls 270 and the motor 250 with the control unit to the motor 250 and control operation of the end effector assembly 100 in accordance with the controls 270, the control unit, and/or other remote control devices, e.g., a footswitch (not shown). The cable 300 further includes one or more wires 310 that connect to an RFID transceiver 290 disposed within the handle housing 210 towards the distal end thereof.

The drive rotor 260 is operably coupled with and extends distally from the motor 250 such that, upon activation of the motor 250, the motor 250 drives rotation of the drive rotor 260. The drive rotor 260 defines a base 262 and the rotor body 264 extending distally from the base 262, which is stationary and surrounds the rotor body 264. The rotor body 264 defines a non-circular cross-sectional configuration, e.g., a square or other polygonal configuration, and is configured for at least partial receipt within the proximally-facing cavity 183e of the proximal driver 182 of the end effector assembly 100 in fixed rotational orientation relative thereto upon engagement of the end effector assembly 100 with the handpiece assembly 200 such that activation of the motor 250 drives rotation of the rotor body 264 of the drive rotor 260 to, in turn, drive the proximal driver 182 of the end effector assembly 100.

The end effector assembly 100 engages with the handpiece assembly 200 in preparation for use of the surgical instrument 10. In order to engage the end effector assembly 100 with the handpiece assembly 200, the end effector assembly 100 is approximated relative to the handpiece assembly 200 such that the retainer cap 170 and the proximal extension portion 164 of the hub housing 161 are inserted into the internal bore 214 of the handle housing 210 of the handpiece assembly 200. As the end effector assembly 100 is approximated in this manner, the grasping ribs 169c of the outer shell 168 of the hub assembly 160 of the end effector assembly 100 are grasped and squeezed inwardly towards one another, thereby causing the upper and lower surfaces of the outer shell 168 to flex outwardly. As the lower surface of the outer shell 168 is flexed outwardly, the engagement finger 169a and the engagement tooth 169b are likewise flexed outwardly. This enables the end effector assembly 100 to be approximated further towards the handpiece assembly 200 such that the engagement tooth 169b is disposed in alignment with and below an engagement aperture 218 defined within the handle housing 210 of the handpiece assembly 200.

Upon release of the grasping ribs 169c of the outer shell 168, the upper and lower surfaces as well as the engagement finger 169a and the engagement tooth 169b are returned inwardly towards their initial positions. In this manner, the engagement tooth 169b is received within the engagement aperture 218 to thereby engage the end effector assembly 100 with the handpiece assembly 200. Disengagement and release of the end effector assembly 100 from the handpiece assembly 200 is affected in the opposite manner.

The end effector assembly 100 is approximated relative to the handpiece assembly 200 to affect the above-detailed engagement, the drive rotor 260 of the handpiece assembly 200 is received within the proximally-facing cavity 183e of the proximal body portion 183a of the proximal driver 182 in fixed rotational orientation thereof, e.g., due to the at least partially complementary configurations thereof. The drive rotor 260, more specifically, is inserted within the proximally-facing cavity 183e and bottoms out therein prior to engagement of the engagement tooth 169b within the engagement aperture 218 and, thus, prior to engagement of the end effector assembly 100 with the handpiece assembly 200. Accordingly, the end effector assembly 100 is required to be further approximated relative to the handpiece assembly 200 in order to affect engagement. As a result, with the rotor body 264 bottomed-out within the proximally-facing cavity 183e, further approximation of the end effector assembly 100 urges the proximal driver 182 distally through and relative to the retainer cap 170, against the bias of the biasing spring 186, to thereby displace the proximally-oriented tab 183d of the external collar 183c of the proximal body portion 183a of the proximal driver 182 from within the distally-oriented notch 148 of the internal collar 176 of the retainer cap 170, thereby rotationally unlocking the proximal and distal drivers 182, 184 from the retainer cap 170 and the hub housing 161. Thus, the inner shaft 140 is unlocked from the outer shaft 120 and permitted to rotate relative thereto.

With the end effector assembly 100 engaged with the handpiece assembly 200 as detailed above, the RFID chip 190 of the end effector assembly 100 is disposed in vertical registration with the RFID transceiver 290 of the handpiece assembly 200, e.g., wherein the RFID transceiver 290 is radially aligned with and disposed radially-outwardly of the RFID chip 190 relative to a longitudinal axis defined through the end effector assembly 100 and the handpiece assembly 200, due to the required orientation of the end effector assembly 100 to enable engagement with the handpiece assembly 200, e.g., such that the engagement tooth 169b is received within the engagement aperture 218. Thus, with the end effector assembly 100 engaged with the handpiece assembly 200, the RFID transceiver 290 may read/write data from/to the memory of the RFID chip 190 and/or communicate read/write data from/to the control unit, e.g., via cable 300.

The data stored in the memory of the RFID chip 190 of the end effector assembly 100, as noted above, may include information of the end effector assembly 100, such as the item number, e.g., SKU number, date of manufacture, manufacture location (e.g., location code), serial number, use count (which may be updated by writing data from the RFID transceiver 290 to the memory of the RFID chip 190), and encryption key(s) to encrypt data, and operating parameters including the home/initial position of the inner shaft 140, the rotation type (rotation versus oscillation), RPM settings (default, high, medium, low), max RPM, pressure setting information, vacuum setting information, outflow setting information, and calibration information. Additional or alternative data may be also saved in the memory of the RFID chip 190.

Continuing with reference to FIGS. 2-6, with the end effector assembly 100 engaged with the handpiece assembly 200 as detailed above, the surgical instrument 10 is ready for use. Based on the operating parameters read from the memory of the RFID chip 190, the surgical instrument 10 may be configured to provide suitable energy to perform the corresponding surgical operation via the end effector assembly 100. In use, the motor 250 of the handpiece assembly 200 is activated to drive rotation of the drive rotor 260. Upon activation of the motor 250, with a head-start or delay relative to activation of the motor 250, or independently thereof, suction is established through the surgical instrument 10, e.g., via activating the vacuum source of the fluid management system.

Activation of the motor 250, in either a rotating or oscillating fashion, drives rotation of the drive rotor 260 which, in turn, drives rotation of the proximal driver 182 to, in turn, drive rotation of the distal driver 184 and thereby rotate or oscillate the inner shaft 140 relative to the outer shaft 120. The rotation or oscillation of the inner shaft 140 relative to the outer shaft 120, together with the suction applied through the inner shaft 140, enables tissue to be drawn into the inner shaft 140, cut, and suctioned, along with fluids and debris, proximally through the inner shaft 140, the drive assembly 180, through output the outflow opening 165 of the proximal extension portion 164 of the hub housing 161, and through the outflow path 220 of the handpiece assembly 200 to outflow the outflow port 400 for output to the collection reservoir of the fluid management system.

Upon engagement of the end effector assembly 100 with the handpiece assembly 200, a control program (not shown) associated with the motor 250 may record the rotational position of drive the rotor 260 as a home position and, after activation, ensure that the drive rotor 260 stops at a rotational position corresponding to the home position, e.g., the closed position of the inner shaft 140 relative to the outer shaft 120. The control program may utilize correlation information, e.g., from the RFID chip 190, correlating, for example, rotation of drive the drive rotor 260 with rotation of the inner shaft 140 to ensure that the inner shaft 140 is returned to the closed position relative to the outer shaft 120 after each activation. Returning to the home position, corresponding to the closed position of the inner shaft 140, also returns the proximal driver 182 to its initial rotational position whereby the proximally-oriented tab 183d of the external collar 183c of the proximal driver 182 is rotationally aligned with the distally-oriented notch 178 of the retainer cap 170. As such, upon disengagement and withdrawal of the end effector assembly 100 from the handpiece assembly 200, the biasing spring 186 returns the proximal driver 182 proximally to thereby the bias proximally-oriented tab 183d into engagement within the distally-oriented notch 178 to re-engage rotational lock rotationally fixing the inner shaft 140 in the closed position relative to the outer shaft 120.

When the end effector assembly 100 is engaged with the handpiece assembly 200, the RFID transceiver 290 is electromagnetically coupled with the RFID chip 190 and able to read data stored in the memory of the RFID chip 190 of the end effector assembly 100. Based on the operating parameters in the read data, the surgical instrument 10 performs a surgical operation via the end effector assembly 100. After completion of the surgical operation, the RFID transceiver 290 may erase the data stored in the memory of the RFID chip 190. Since there is no data left in the memory of the RFID chip 190, the end effector assembly 100 cannot be reused when the end effector assembly 100 is connected to the handpiece assembly 200 again. In this way, after completion of the surgical operation, the disposable end effector assembly 100 cannot be re-used.

In an aspect, the RFID chip 190 may include an irreversible component, which prevents re-use of the end effector assembly 100. The irreversible component may be activated after completion of the surgical operation. That is, after completion of the surgical operation, the RFID transceiver 290 may activate the irreversible component, which then irreversibly deactivates the RFID chip 190. The irreversible component may be an electrical fuse, and when activated, may be melted and separated into two pieces, thereby disconnecting electrical coupling with the RFID transceiver 290.

In another aspect, the RFID chip 190 may be disposed or cannot be re-used when the memory thereof is activated. After completion of the surgical operation, the RFID transceiver 290 activates the memory of the RFID chip 190 by writing new data on the memory and immediately reading the written data from the memory.

Deactivating the end effector assembly 100 may be enabled with the methods described above, singly or in any combination. Furthermore, other methods for deactivating the RFID chip 190, which are readily contemplated by persons killed in the art, are incorporated in this disclosure.

Referring back to FIG. 2, as an alternative to the handpiece assembly 200 configured for manual grasping and manipulation during use, the surgical instrument 10 may alternatively be configured for use with a robotic surgical system wherein the handle housing 210 is configured to engage a robotic arm of the robotic surgical system. The robotic surgical system may employ various robotic elements to assist the surgeon and allow remote operation (or partial remote operation). More specifically, various robotic arms, gears, cams, pulleys, electric and mechanical motors, etc. may be employed for this purpose and may be designed with the robotic surgical system to assist the surgeon during the course of an operation or treatment. The robotic surgical system may include remotely steerable systems, automatically flexible surgical systems, remotely flexible surgical systems, remotely articulating surgical systems, wireless surgical systems, modular or selectively configurable remotely operated surgical systems, etc.

The robotic surgical system may be employed with one or more consoles that are next to the operating theater or located in a remote location. In this instance, one team of surgeons or nurses may prep the patient for surgery and configure the robotic surgical system with the surgical device disclosed herein while another surgeon (or group of surgeons) remotely controls the surgical device via the robotic surgical system. As can be appreciated, a highly skilled surgeon may perform multiple operations in multiple locations without leaving his/her remote console which can be both economically advantageous and a benefit to the patient or a series of patients.

The robotic arms of the robotic surgical system are typically coupled to a pair of master handles by a controller. The handles can be moved by the surgeon to produce a corresponding movement of the working ends of any type of surgical instrument (e.g., end effectors, graspers, knifes, scissors, cameras, fluid delivery devices, etc.) which may complement the use of the tissue resecting devices described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

The invention may be described by reference to the following numbered paragraphs:-
1. A surgical device to perform a surgical operation, the surgical device comprising:
   a limited-use component configured to perform a surgical operation, wherein the limited-use component can be used for a pre-determined number of uses and includes:
      a body forming a pocket; and
      a radio frequency identification (RFID) tag disposed within the pocket; and
   a reusable component operationally coupled to the limited-use component, the reusable component including an RFID tag transceiver configured to read operating parameters from a memory of the RFID tag of the limited-use component,
   wherein the operating parameters are erased from the memory when the limited-use component has been used for the pre-determined number of uses.
2. The surgical device according to paragraph 1, wherein the RFID tag transceiver is disposed in vertical registration with the RFID tag when the reusable component is operationally coupled to the limited-use component.
3. The surgical device according to paragraph 1, wherein the operating parameters are encrypted to limit reproduction and reprograming of the operating parameters.
4. The surgical device according to paragraph 1, wherein the operating parameters include at least one of a home/initial position of the limited-use component, a rotation type, revolution per minute (RPM) settings, a maximum RPM, pressure setting information, vacuum setting information, outflow setting information, or calibration information.
5. A surgical device to perform a surgical operation, the surgical device comprising:
   a limited-use component configured to perform a surgical operation, wherein the limited-use component can be used for a pre-determined number of uses and includes:
      a body forming a pocket; and
      a radio frequency identification (RFID) tag disposed within the pocket; and
   a reusable component operationally coupled to the limited-use component, the reusable component including an RFID tag transceiver configured to read operating parameters from a memory of the RFID tag of the limited-use component,
   wherein the limited-use component cannot be reused after the memory of the RFID tag is activated.
6. The surgical device according to paragraph 5, wherein activation of the memory includes: the RFID tag transceiver writing new information in the memory and immediately reading the new information from the memory.
7. The surgical device according to paragraph 5, wherein the RFID tag transceiver is disposed in vertical registration with the RFID tag when the reusable component is operationally coupled to the limited-use component.
8. The surgical device according to paragraph 7, wherein the operating parameters are encrypted to limit reproduction and reprograming of the operating parameters.
9. The surgical device according to paragraph 7, wherein the operating parameters include at least one of a home/initial position of the limited-use component, a rotation type, revolution per minute (RPM) settings, a maximum RPM, pressure setting information, vacuum setting information, outflow setting information, or calibration information.
10. A surgical device to perform a surgical operation, the surgical device comprising:
   a limited-use component configured to perform a surgical operation, wherein the limited-use component can be used for a pre-determined number of uses and includes:
      a body forming a pocket;
      a radio frequency identification (RFID) tag disposed within the pocket; and
      an irreversible electrical component electrically coupled to the RFID tag; and
   a reusable component operationally coupled to the limited-use component, the reusable component including an RFID tag transceiver configured to read operating parameters from a memory of the RFID tag of the limited-use component.
11. The surgical device according to paragraph 10, wherein the irreversible electrical component is activated when the limited-use component has been used for the predetermined number of uses.
12. The surgical device according to paragraph 10, wherein the irreversible electrical component is a fuse.
13. The surgical device according to paragraph 10, wherein the RFID tag transceiver is disposed in vertical registration with the RFID tag when the reusable component is operationally coupled to the limited-use component.
14. The surgical device according to paragraph 10, wherein the operating parameters are encrypted to limit reproduction and reprograming of the operating parameters.
15. The surgical device according to paragraph 10, wherein the operating parameters include at least one of a home/initial position of the limited-use component, a rotation type, revolution per minute (RPM) settings, a maximum RPM, pressure setting information, vacuum setting information, outflow setting information, or calibration information.

## Claims

1. A surgical device to perform a surgical operation, the surgical device comprising:
a limited-use component configured to perform a surgical operation, wherein the limited-use component can be used for a pre-determined number of uses and includes:
a body forming a pocket; and
a radio frequency identification (RFID) tag disposed within the pocket; and
a reusable component operationally coupled to the limited-use component, the reusable component including an RFID tag transceiver configured to read operating parameters from a memory of the RFID tag of the limited-use component,
wherein the operating parameters are erased from the memory when the limited-use component has been used for the pre-determined number of uses.

2. The surgical device according to claim 1, wherein the RFID tag transceiver is disposed in vertical registration with the RFID tag when the reusable component is operationally coupled to the limited-use component.

3. The surgical device according to claim lor 2, wherein the operating parameters are encrypted to limit reproduction and reprograming of the operating parameters.

4. The surgical device according to claim 1, 2 or 3 wherein the operating parameters include at least one of a home/initial position of the limited-use component, a rotation type, revolution per minute (RPM) settings, a maximum RPM, pressure setting information, vacuum setting information, outflow setting information, or calibration information.

5. A surgical device to perform a surgical operation, the surgical device comprising:
a limited-use component configured to perform a surgical operation, wherein the limited-use component can be used for a pre-determined number of uses and includes:
a body forming a pocket; and
a radio frequency identification (RFID) tag disposed within the pocket; and
a reusable component operationally coupled to the limited-use component, the reusable component including an RFID tag transceiver configured to read operating parameters from a memory of the RFID tag of the limited-use component,
wherein the limited-use component cannot be reused after the memory of the RFID tag is activated.

6. The surgical device according to claim 5, wherein activation of the memory includes: the RFID tag transceiver writing new information in the memory and immediately reading the new information from the memory.

7. The surgical device according to claim 5 or 6, wherein the RFID tag transceiver is disposed in vertical registration with the RFID tag when the reusable component is operationally coupled to the limited-use component.

8. The surgical device according to claim 7, wherein the operating parameters are encrypted to limit reproduction and reprograming of the operating parameters.

9. The surgical device according to claim 7 or 8, wherein the operating parameters include at least one of a home/initial position of the limited-use component, a rotation type, revolution per minute (RPM) settings, a maximum RPM, pressure setting information, vacuum setting information, outflow setting information, or calibration information.

10. A surgical device to perform a surgical operation, the surgical device comprising:
a limited-use component configured to perform a surgical operation, wherein the limited-use component can be used for a pre-determined number of uses and includes:
a body forming a pocket;
a radio frequency identification (RFID) tag disposed within the pocket; and
an irreversible electrical component electrically coupled to the RFID tag; and
a reusable component operationally coupled to the limited-use component, the reusable component including an RFID tag transceiver configured to read operating parameters from a memory of the RFID tag of the limited-use component.

11. The surgical device according to claim 10, wherein the irreversible electrical component is activated when the limited-use component has been used for the predetermined number of uses.

12. The surgical device according to claim 10 or 11, wherein the irreversible electrical component is a fuse.

13. The surgical device according to claim 10, 11 or 12 wherein the RFID tag transceiver is disposed in vertical registration with the RFID tag when the reusable component is operationally coupled to the limited-use component.

14. The surgical device according to any one of claims 10 to 13, wherein the operating parameters are encrypted to limit reproduction and reprograming of the operating parameters.

15. The surgical device according to any one of claims 10 to 14, wherein the operating parameters include at least one of a home/initial position of the limited-use component, a rotation type, revolution per minute (RPM) settings, a maximum RPM, pressure setting information, vacuum setting information, outflow setting information, or calibration information.
